# EUROPÄISCHE PATENTSCHRIFT

(11) **EP 3 749 196 B1**
(45) Veröffentlichungstag und Bekanntmachung des Hinweises auf die Patenterteilung: **03.04.2024**
(21) Anmeldenummer: 19704289.8
(22) Anmeldetag: 06.02.2019
(51) Int. Cl.: A61B 5/055, A61B 18/08, A61B 18/12, A61B 18/14

(54) **HYBRIDSYSTEM FÜR DIE DURCHFÜHRUNG EINER MAGNETRESONANZTOMOGRAPHIE UND EINER RADIOFREQUENZABLATION SOWIE VERFAHREN ZU DESSEN BETRIEB**
HYBRID SYSTEM FOR PERFORMING A MAGNETIC RESONANCE TOMOGRAPHY AND A RADIOFREQUENCY ABLATION, AND METHOD FOR THE OPERATION THEREOF
SYSTÈME HYBRIDE POUR LA RÉALISATION D'UNE IMAGERIE PAR RÉSONANCE MAGNÉTIQUE ET D'UNE ABLATION PAR FRÉQUENCE RADIO AINSI QUE PROCÉDÉ DE FONCTIONNEMENT

(30) Priorität: 09.02.2018 DE 102018102965
(43) Veröffentlichungstag der Anmeldung: 16.12.2020
(73) Patentinhaber: Otto-von-Guericke-Universität Magdeburg, 39106 Magdeburg (DE)
(72) Erfinder: GERLACH, Thomas, 39128 Magdeburg (DE); PANNICKE, Enrico, 39114 Magdeburg (DE)
(74) Vertreter: Kröncke, Rolf
(86) Internationale Anmeldenummer: PCT/EP2019/052896
(87) Internationale Veröffentlichungsnummer: WO 2019/154850

(56) Entgegenhaltungen:
- US-A- 5 323 778
- US-A1- 2011 301 450
- THOMAS GERLACH ET AL: "Proof of Concept of a Hybrid MRI-Ablation System", ABSTRACT BOOK 3RD CONFERENCE ON IMAGE-GUIDED INTERVENTIONS & FOKUS NEURORADIOLOGIE IMAGE-GUIDED INTERVENTIONS & FOKUS NEURORADIOLOGIE RECENT PROGRESS AND DEVELOPMENTS, 6. November 2017 (2017-11-06), Seite 15, XP55580789, Magdeburg, Germany
- HUE YIK-KIONG ET AL: "Magnetic Resonance Mediated Radiofrequency Ablation", IEEE TRANSACTIONS ON MEDICAL IMAGING, IEEE SERVICE CENTER, PISCATAWAY, NJ, US, Bd. 37, Nr. 2, 1. Februar 2018 (2018-02-01), Seiten 417-427, XP011676591, ISSN: 0278-0062, DOI: 10.1109/TMI.2017.2753739 [gefunden am 2018-02-01]
- KIM SHULTZ ET AL: "Tissue Impedance Implications of Performing RF Ablation at 64 MHz Temperature Impedance", PROC. INTL. SOC. MAG. RESON. MED., 1. Januar 2012 (2012-01-01), Seite 559, XP55580812,

## Beschreibung

Die Erfindung betrifft ein Hybridsystem für die Durchführung einer Magnetresonanztomographie (MRT) und einer Radiofrequenzablation an einem Patienten. Die Erfindung betrifft außerdem ein Verfahren zum Betrieb eines derartigen Hybridsystems.

Die Magnetresonanztomographie, abgekürzt MRT, ist ein bildgebendes Verfahren, das in der Medizin zur Darstellung von Struktur und Funktion von Gewebe und Organen im Körper eingesetzt wird. Mit der MRT kann man Schnittbilder des Körpers eines Patienten erzeugen. Zur Durchführung der MRT werden vom Magnetresonanztomographiesystem Hochfrequenzsignale erzeugt, die nachfolgend als MRT-Hochfrequenzsignale bezeichnet werden. Diese MRT-Hochfrequenzsignale werden bspw. über eine Sendespule in Form von magnetischen Wechselfeldern mit hoher Amplitude in den Patienten eingespeist. Bei einer bestimmten Frequenz (der sog. Larmorfrequenz) werden hierdurch bestimmte Atomkerne im Körper resonant angeregt, wodurch nach Ausschalten des HF-Feldes in einem Empfängerstromkreis ein elektrisch-induziertes Signal gemessen werden kann.

Die Prozedur aus Senden von HF-Feldern und Empfangen von Messsignalen werden mit Einschalten von separaten ortsabhängigen Magnetfeldern wiederholt. Dies ermöglicht eine Ortskodierung der Signale im Körper. Die aufgenommenen Signale können dann in einem rechnergestützten Post-Processing ausgewertet und visualisiert werden.

Die Radiofrequenzablation, abgekürzt RFA, ist ein hyperthermischer und minimal-invasiver Ansatz zur Zerstörung von Tumoren und Metastasen. Die Radiofrequenzablation wird in der Regel unter Nutzung medizinischer Bildgebung durchgeführt, wozu bevorzugt die Magnetresonanztomographie genutzt wird. Bei der Radiofrequenzablation wird ein Hochfrequenzgenerator über abgeschirmte Kabel mit einer Ablationselektrode gekoppelt, über die die Radiofrequenzenergie in den Patienten eingespeist wird. Hierbei ist häufig ein großer Aufwand erforderlich, um eine nachteilige Beeinflussung der MRT-Bildgebung durch die Radiofrequenzablation zu vermeiden. Insgesamt ist der hierfür erforderliche apparative Aufwand sehr hoch.

Ein entsprechender Vorschlag geht bereits aus der Veröffentlichung von Thomas Gerlach et.al.: "Proof of Concept of a Hybrid MRI-Ablation System", Abstract Book 3rd Conference on Image-Guided Interventions & Fokus Neuroradiologie Image-Guided Interventions & Fokus Neuroradiologie Recent Progress and Developments, 6. November 2017 (2017-11-06), Seite 15 hervor. Aus der US 2011/0301450 A1 ist eine kombinierte Magnetresonanztomographie und Radiofrequenzablation bekannt. Aus der Veröffentlichung von Hue Yik-Kiong et.al.: "Magnetic Resonance Mediated Radiofrequency Ablation", IEEE Transactions on Medical Imaging, IEEE Service Center, Piscataway, NJ, US, Bd. 37, Nr. 2, 1. Februar 2018 (2018-02-01), Seiten 417-427 ist ein vergleichbares System bekannt. Aus der US 5,323,778 sind Verfahren und Vorrichtungen für die Magnetresonanztomographie und der Erwärmung von Gewebe bekannt. Aus der Veröffentlichung von Kim Schultz et.al.: "Tissue Impedance Implications of Performing RF Ablation at 64 MHz Temperature Impedance", Proc. Intl. Soc. Mag. Reson. Med., 1. Januar 2012 (2012-01-01), Seite 559 sind Vorschläge für die Durchführung einer Radiofrequenzablation bei 64 MHz bekannt.

Der Erfindung liegt die Aufgabe zugrunde, den Aufwand für die MRT-geführte Radiofrequenzablation zu verringern.

Diese Aufgabe wird gelöst durch ein Hybridsystem für die Durchführung einer Magnetresonanztomographie (MRT) und einer Radiofrequenzablation an einem Patienten gemäß Anspruch 1. Dies beinhaltet:
a) das Hybridsystem weist ein Magnetresonanztomographiesystem auf, in dem MRT-Hochfrequenzsignale zur Durchführung der Magnetresonanztomographie erzeugbar und an einem Ausgangsanschluss des Magnetresonanztomographiesystems bereitstellbar sind,
b) das Hybridsystem weist wenigstens eine Ablationselektrode zur Durchführung der Radiofrequenzablation auf,
c) die wenigstens eine Ablationselektrode ist mit dem Ausgangsanschluss des Magnetresonanztomographiesystems gekoppelt, sodass die Radiofrequenzablation über die wenigstens eine Ablationselektrode mittels der MRT-Hochfrequenzsignale durchführbar ist.

Es wurde festgestellt, dass die vom Magnetresonanztomographiesystem bereitgestellten MRT-Hochfrequenzsignale, die für die Durchführung der Magnetresonanztomographie und deren Bildgebung eingesetzt werden, auch für die Durchführung der Radiofrequenzablation geeignet sind. Insbesondere ist die bspw. am Ausgang eines HF-Verstärkers des Magnetresonanztomographiesystems bereitgestellte Energie der MRT-Hochfrequenzsignale ausreichend für die Durchführung einer Radiofrequenzablation. Somit kann das gesamte System vereinfacht werden, da kein gesonderter Hochfrequenzgenerator für die Speisung der Ablationselektrode erforderlich ist. Stattdessen kann die Ablationselektrode oder gegebenenfalls mehrere Ablationselektroden direkt oder über Zusatzbauelemente am Ausgangsanschluss des Magnetresonanztomographiesystems angeschlossen werden. Auf diese Weise wird die wenigstens eine Ablationselektrode mit der Hochfrequenzenergie der MRT-Hochfrequenzsignale gespeist, sodass direkt mittels der MRT-Hochfrequenzsignale die Radiofrequenzablation durchgeführt werden kann.

Auf diese Weise wird das gesamte System vereinfacht, es können Kosten gespart werden und zudem sind weniger Komponenten erforderlich, die geprüft werden müssen oder die ausfallen oder Störungen verursachen könnten. Zudem können Störeinflüsse der Radiofrequenzablation bei der MRT-Bildgebung reduziert werden, da keine gesonderten, nicht mit den MRT-Hochfrequenzsignalen synchronisierten Signale in den Patienten eingespeist werden.

Gemäß einer vorteilhaften Weiterbildung der Erfindung ist vorgesehen, dass das Hybridsystem eine Pulserzeugungsschaltung aufweist, durch die der wenigstens einen Ablationselektrode die für die Radiofrequenzablation erforderlichen Hochfrequenzsignale gepulst zuführbar sind. Auf diese Weise kann eine gepulste Radiofrequenzablation durchgeführt werden. Insbesondere in Kombination mit der Nutzung der MRT-Hochfrequenzsignale für die Radiofrequenzablation ergibt sich der vorteilhafte Synergieeffekt, dass die MRT-Hochfrequenzsignale bei üblichen Magnetresonanztomographiesystemen bereits gepulst erzeugt werden, z.B. in Form von Pulszügen, die aus einer Vielzahl von einzelnen Hochfrequenzpulsen bestehen, wobei zwischen solchen Pulszügen eine Pause vorhanden ist, in der keine Hochfrequenzpulse erzeugt werden.

Gemäß einer vorteilhaften Weiterbildung der Erfindung ist vorgesehen, dass die wenigstens eine Ablationselektrode mit einem Hochfrequenzsignal mit der Larmorfrequenz gespeist ist. Dies hat den Vorteil, dass der von der Ablationselektrode in den Patienten eingespeiste Ablationsstrom ein magnetisches Wirbelfeld erzeugt, welches wiederum messbare Magnetresonanz-Signale generiert, sodass auch dieses Wirbelfeld durch die MRT-Bildgebung erfasst und visualisiert werden kann.

Gemäß einer vorteilhaften Weiterbildung der Erfindung ist vorgesehen, dass das Hybridsystem dazu eingerichtet ist, den durch die Ablationselektrode in den Patienten eingespeisten Ablationsstrom über die Bildgebung des Magnetresonanztomographiesystems aufzunehmen und zu visualisieren. Dies hat den Vorteil, dass dem Anwender des Hybridsystems zusätzliche Informationen über den aktuellen Zustand der Radiofrequenzablation gegeben werden können. Es können z.B. die bei der MRT-Bildgebung resultierenden Signalintensitäten qualitative Auskünfte über den Stromverlauf des Ablationsstroms liefern. Für die Stromvisualisierung kann z.B. eine Messung der Amplitude und Phase der Magnetfelder durchgeführt werden, woraus die Amplitude und Phase des Ablationsstroms rekonstruiert werden können.

Durch die Nutzung der MRT-Hochfrequenzsignale für die Radiofrequenzablation ist zudem keine aufwendige Phasen-Synchronisation zwischen dem Ablationssignal und der MRT-Bildgebung erforderlich.

Gemäß der Erfindung ist vorgesehen, dass das Hybridsystem dazu eingerichtet ist, die MRT-Hochfrequenzsignale entweder der wenigstens einen Ablationselektrode oder einer MRT-Sendespule des Magnetresonanztomographiesystems zuzuführen. Auf diese Weise wird eine gemischte Nutzung der einzelnen Signalpulse der MRT-Hochfrequenzsignale vermieden. Stattdessen werden die MRT-Hochfrequenzsignale zu einem Zeitpunkt immer nur einer Nutzung zugeführt, d.h. entweder der Radiofrequenzablation oder der Bildgebung der Magnetresonanztomographie. Hierdurch kann eine hohe Bildqualität bei der Magnetresonanztomographie sichergestellt werden. Für die Zuführung der MRT-Hochfrequenzsignale entweder zur Ablationselektrode oder zur MRT-Sendespule kann bspw. ein computergesteuerter Umschalter vorhanden sein.

Die eingangs genannte Aufgabe wird ferner gelöst durch ein Verfahren gemäß Anspruch 5 zum Betrieb eines Hybridsystems der zuvor erläuterten Art, bei dem der wenigstens einen Ablationselektrode zumindest zeitweise die MRT-Hochfrequenzsignale des Magnetresonanztomographiesystems zugeführt werden. Auch hierdurch lassen sich die zuvor erläuterten Vorteile realisieren.

Gemäß der Erfindung ist vorgesehen, dass die MRT-Hochfrequenzsignale des Magnetresonanztomographiesystems wechselweise der wenigstens einen Ablationselektrode und einer MRT-Sendespule des Magnetresonanztomographiesystems zugeführt werden. Auf diese Weise kann jeweils ein ungestörtes Signal mit voller Signalintensität entweder für die eine Anwendung oder die andere Anwendung genutzt werden. Dies kommt insbesondere der Qualität der MRT-Bildgebung zugute.

Gemäß einer vorteilhaften Weiterbildung der Erfindung ist vorgesehen, dass die Bilderzeugung für die Visualisierung der Magnetresonanztomographie unterbrochen wird, während die wenigstens eine Ablationselektrode mit den MRT-Hochfrequenzsignalen gespeist wird. Diese Unterbrechung der Bilderzeugung ist für den Anwender nicht störend, da sie derart kurzfristig ist, dass sie im Wesentlichen nicht wahrgenommen wird.

Gemäß einer vorteilhaften Weiterbildung der Erfindung ist vorgesehen, dass das vom Ablationsstrom der wenigstens einen Ablationselektrode im Patienten erzeugte magnetische Wirbelfeld über das Magnetresonanztomographiesystem aufgenommen und als Stromverlauf visualisiert wird. Auf diese Weise können dem Anwender zusätzliche Informationen über den aktuellen Zustand der Radiofrequenzablation geliefert werden.

Die Erfindung wird nachfolgend anhand von Ausführungsbeispielen unter Verwendung von Zeichnungen näher erläutert.

Es zeigen
- Figur 1: ein Hybridsystem in schematisierter Darstellung und
- Figur 2: die Erzeugung gepulster Hochfrequenzsignale und
- Figuren 3, 4: weitere Ausführungsformen eines Hybridsystems.

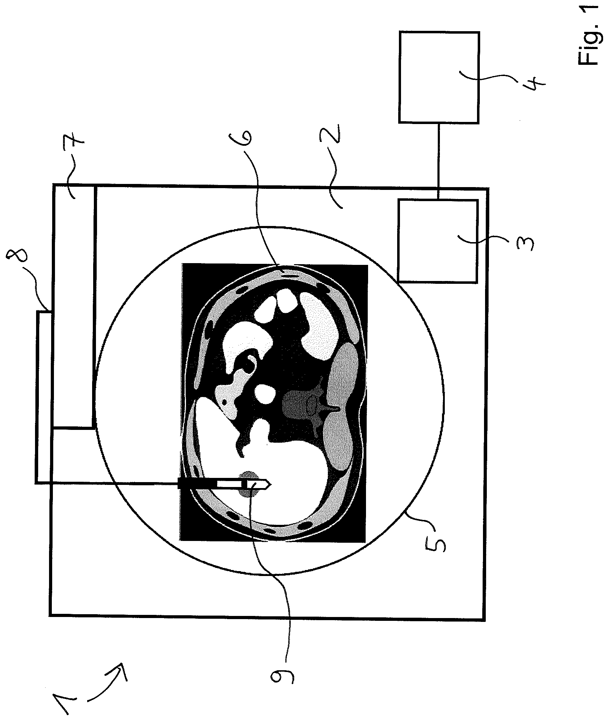

Die Figur 1 zeigt ein Hybridsystem 1, das ein Magnetresonanztomographiesystem 2 aufweist. Das Magnetresonanztomographiesystem 2 kann von üblicher, bekannter Bauart sein. Das Magnetresonanztomographiesystem 2 weist bspw. eine für Magnetresonanztomographie-Untersuchungen zu nutzende Röhre 5 auf, in der ein Patient 6 platziert werden kann. Über bspw. in der Wandung der Röhre 5 angeordnete Sendespulen werden die von einem Hochfrequenz-Verstärker 7 des Magnetresonanztomographiesystems 2 bereitgestellten MRT-Hochfrequenzsignale auf den Patienten 6 übertragen. Die resultierenden, empfangsseitig aufgenommenen Signale des Magnetresonanztomographiesystems werden über eine Bildgebung 3 des Magnetresonanztomographiesystems 2 erfasst und verarbeitet. Die hierdurch erzeugten Bildinformationen können auf einem Bildanzeigegerät 4 dargestellt werden.

Das Hybridsystem 1 ist ferner zur Durchführung einer Radiofrequenzablation an dem Patienten 6 eingerichtet. Hierzu ist zumindest eine Ablationselektrode 9 vorhanden, die bspw. an einem zu entfernenden Tumor im Patienten 6 platziert werden kann. Die Ablationselektrode 9 ist über eine Leitung mit einem Ausgangsanschluss 8 des Magnetresonanztomographiesystems, z.B. einem Ausgangsanschluss des HF-Ausgangsverstärkers 7, verbunden. Auf diese Weise werden die am Ausgangsanschluss 8 bereitgestellten MRT-Hochfrequenzsignale der Ablationselektrode 9 zugeführt und in den Patienten 6 eingespeist.

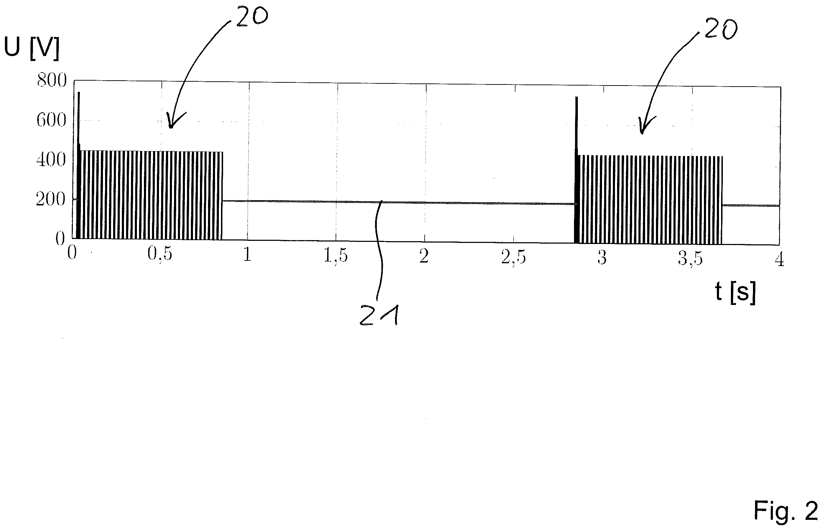

Die Figur 2 zeigt einen beispielhaften Zeitverlauf der MRT-Hochfrequenzsignale 20. Die MRT-Hochfrequenzsignale 20 können in Form von einzelnen Pulszügen, von denen in der Figur 2 zwei Pulszüge dargestellt sind, erzeugt werden. Ein Pulszug besteht aus einer Vielzahl von einzelnen Hochfrequenz-Pulsen. Zwischen den einzelnen Pulszügen ist eine Pause, im dargestellten Beispiel z.B. in einer Länge von ca. 2 Sekunden. Durch die Linie 21 wird eine mittlere Spannung dargestellt, die sich als effektive Spannung für den Ablationsprozess an der Ablationselektrode 9 einstellt. Beispielsweise kann der erste in Figur 2 dargestellte Pulszug 20 für die Radiofrequenzablation genutzt werden und dementsprechend nur der Ablationselektrode zugeführt werden und der andere, zweite dargestellte Pulszug 20 für die Bildgebung der Magnetresonanztomographie, d.h. dieser Pulszug wird lediglich einer MRT-Sendespule zugeführt.

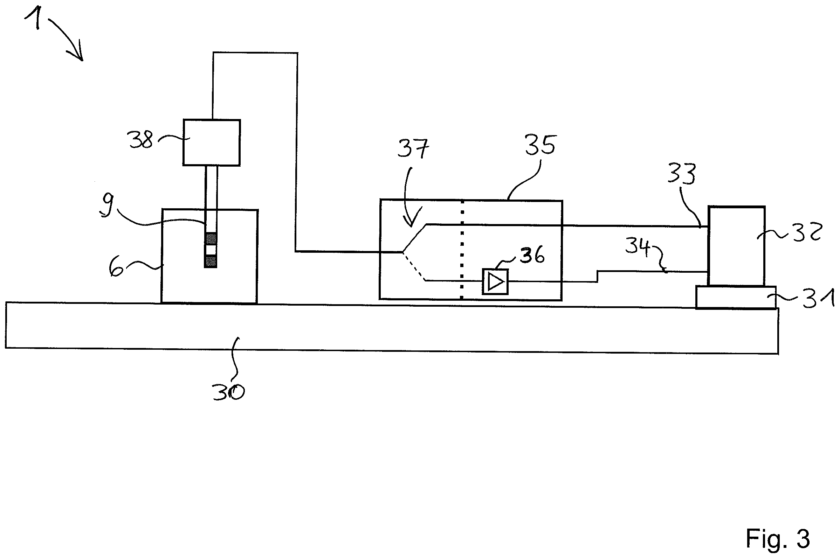

Die Figur 3 zeigt weitere Merkmale des Hybridsystems 1, die z.B. in dem anhand der Figur 1 erläuterten Hybridsystem realisiert sein können. Erkennbar ist eine Patientenliege 30, auf der der Patient 6 platziert ist. Dargestellt ist ferner auch wiederum die Ablationselektrode 9. Die Ablationselektrode 9 ist über eine Anpassungsschaltung 38 mit einem Funktionsblock 35 verbunden. Der Funktionsblock 35 enthält einen Umschalter 37, z.B. in Form eines RX-TX-Switch. Über den Umschalter 37 kann die Ablationselektrode 9 wahlweise mit einem Sendekanal oder einem Empfangskanal des Hybridsystems 1 verbunden werden. Der Sendekanal kann über eine Sendeleitung 33 mit dem Funktionsblock 35 verbunden sein, der Empfangskanal über eine Empfangsleitung 34. Der Funktionsblock 35 weist für den Empfangskanal einen Vorverstärker 36 auf, der mit der Empfangsleitung 34 verbunden ist. Die Sendeleitung 33 und die Empfangsleitung 34 sind mit einem Spulenstecker 32 verbunden, an den der Sendekanal und der Empfangskanal des Hybridsystems 1 angeschlossen werden können. Der Spulenstecker 32 kann mit einem Spulenterminal 31 verbunden sein, an dem die Hochfrequenzsignale für die Bildgebung bzw. für die Ablation entnommen werden können.

Im Betrieb des Hybridsystems gemäß Figur 3 wird computergesteuert der Umschalter 37 umgeschaltet, z.B. bei den in Figur 2 dargestellten Pulszügen abwechselnd, sodass die MRT-Hochfrequenzsignale wechselweise der einen oder der anderen Anwendung zugeführt werden, sodass die Ablationselektrode 9 wechselweise im Sendefall oder im Empfangsfall fungiert.

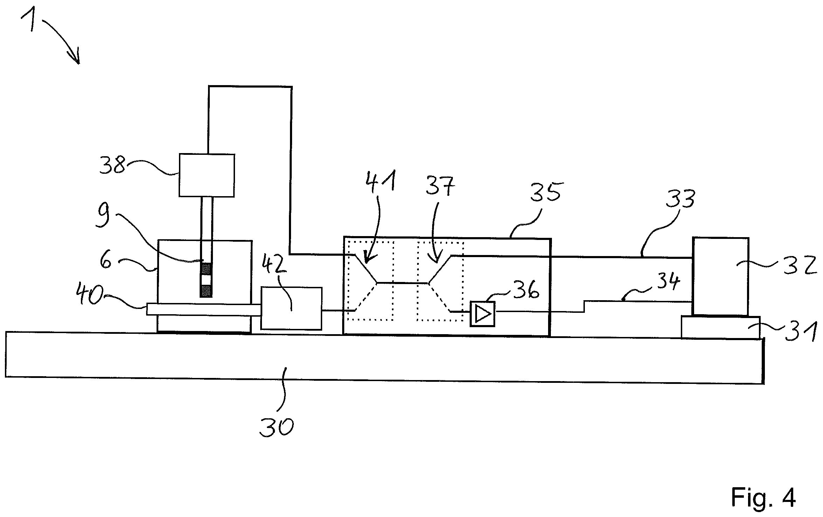

Die Figur 4 zeigt eine weitere Ausgestaltung des Hybridsystems 1, die sich von der Ausführungsform der Figur 3 wie folgt unterscheidet. Für die MRT-Bildgebung bzw. die Aufnahme der Magnetfelder zur Durchführung der MRT-Bildgebung ist eine MRT-Spule 40 vorhanden, z.B. in Form einer Leiterschleife. Die MRT-Spule 40 ist über eine Anpassungsschaltung 42 mit dem Funktionsblock 35 verbunden. Der Funktionsblock 35 weist außer dem bereits erläuterten Umschalter 37 und dem Vorverstärker 36 zusätzlich einen weiteren Umschalter 41 auf.

Im Betrieb des Hybridsystems gemäß Figur 4 wird computergesteuert der Umschalter 41 umgeschaltet, z.B. bei den in Figur 2 dargestellten Pulszügen abwechselnd, sodass die MRT-Hochfrequenzsignale wechselweise der Ablationselektrode 9 oder der MRT-Spule 40 zugeführt werden.

Beim Umschalten mit dem Umschalter 41 auf die Ablationselektrode 9 wäre die Anwendung Ablation möglich und beim Umschalten auf die MRT-Spule 40 wäre die Anwendung MRT-Bildgebung möglich.

## Patentansprüche

1. Hybridsystem (1) für die Durchführung einer Magnetresonanztomographie (MRT) und einer Radiofrequenzablation an einem Patienten (6), mit folgenden Merkmalen:
a) das Hybridsystem (1) weist ein Magnetresonanztomographiesystem (2) auf, in dem MRT-Hochfrequenzsignale (20) zur Durchführung der Magnetresonanztomographie erzeugbar und an einem Ausgangsanschluss (8) des Magnetresonanztomographiesystems (2) bereitstellbar sind,
b) das Hybridsystem (1) weist wenigstens eine Ablationselektrode (9) zur Durchführung der Radiofrequenzablation auf,
c) die wenigstens eine Ablationselektrode (9) ist mit dem Ausgangsanschluss (8) des Magnetresonanztomographiesystems (2) gekoppelt, sodass die Radiofrequenzablation über die wenigstens eine Ablationselektrode (9) mittels der MRT-Hochfrequenzsignale (20) durchführbar ist,
**dadurch gekennzeichnet, dass** das Hybridsystem (1) dazu eingerichtet ist, die MRT-Hochfrequenzsignale (20) entweder der wenigstens einen Ablationselektrode (9) oder einer MRT-Sendespule des Magnetresonanztomographiesystems zuzuführen.

2. Hybridsystem nach Anspruch 1, **dadurch gekennzeichnet, dass** das Hybridsystem (1) eine Pulserzeugungsschaltung aufweist, durch die der wenigstens einen Ablationselektrode (9) die für die Radiofrequenzablation erforderlichen Hochfrequenzsignale gepulst zuführbar sind.

3. Hybridsystem nach einem der vorhergehenden Ansprüche, **dadurch gekennzeichnet, dass** die wenigstens eine Ablationselektrode (9) mit einem Hochfrequenzsignal mit der Larmorfrequenz gespeist ist.

4. Hybridsystem nach einem der vorhergehenden Ansprüche, **dadurch gekennzeichnet, dass** das Hybridsystem (1) dazu eingerichtet ist, den durch die Ablationselektrode (9) in den Patienten (6) eingespeisten Ablationsstrom über die Bildgebung (3) des Magnetresonanztomographiesystems (2) aufzunehmen und zu visualisieren.

5. Verfahren zum Betrieb eines Hybridsystems nach einem der vorhergehenden Ansprüche, **dadurch gekennzeichnet, dass** der wenigstens einen Ablationselektrode (9) zumindest zeitweise die MRT-Hochfrequenzsignale (20) des Magnetresonanztomographiesystems (2) zugeführt werden, **dadurch gekennzeichnet, dass** die MRT-Hochfrequenzsignale (20) des Magnetresonanztomographiesystems (2) wechselweise der wenigstens einen Ablationselektrode (9) und einer MRT-Sendespule des Magnetresonanztomographiesystems (2) zugeführt werden.

6. Verfahren nach Anspruch 5, **dadurch gekennzeichnet, dass** die Bilderzeugung für die Visualisierung der Magnetresonanztomographie unterbrochen wird, während die wenigstens eine Ablationselektrode (9) mit den MRT-Hochfrequenzsignalen (20) gespeist wird.

7. Verfahren nach einem der Ansprüche 5 bis 6, **dadurch gekennzeichnet, dass** das vom Ablationsstrom der wenigstens einen Ablationselektrode (9) im Patienten (6) erzeugte magnetische Wirbelfeld über das Magnetresonanztomographiesystem (2) aufgenommen und als Stromverlauf visualisiert wird.

## Claims

1. Hybrid system (1) for performing magnetic resonance imaging (MRI) and radiofrequency ablation on a patient (6), with the following features:
a) the hybrid system (1) has a magnetic resonance imaging system (2) in which MRI high-frequency signals (20) can be generated for carrying out the magnetic resonance imaging and can be made available at an output connection (8) of the magnetic resonance imaging system (2),
b) the hybrid system (1) has at least one ablation electrode (9) for performing radiofrequency ablation,
c) the at least one ablation electrode (9) is coupled to the output connection (8) of the magnetic resonance imaging system (2), so that the radiofrequency ablation can be performed via the at least one ablation electrode (9) by means of the MRI high-frequency signals (20),
**characterized in that** the hybrid system (1) is set up to supply the MRI high-frequency signals (20) either to the at least one ablation electrode (9) or to an MRI transmitting coil of the magnetic resonance imaging system.

2. Hybrid system according to claim 1, **characterized in that** the hybrid system (1) has a pulse generation circuit by means of which the high-frequency signals required for radiofrequency ablation can be supplied in pulsed form to the at least one ablation electrode (9).

3. Hybrid system according to one of the preceding claims, **characterized in that** the at least one ablation electrode (9) is supplied with a high-frequency signal at the Larmor frequency.

4. Hybrid system according to one of the preceding claims, **characterized in that** the hybrid system (1) is set up to record and visualize the ablation current fed into the patient (6) by the ablation electrode (9) via the imaging (3) of the magnetic resonance imaging system (2).

5. Method for operating a hybrid system according to one of the preceding claims, **characterized in that** the MRI high-frequency signals (20) of the magnetic resonance imaging system (2) are supplied at least intermittently to the at least one ablation electrode (9), **characterized in that** the MRI high-frequency signals (20) of the magnetic resonance imaging system (2) are supplied alternately to the at least one ablation electrode (9) and to an MRI transmission coil of the magnetic resonance imaging system (2).

6. Method according to claim 5, **characterized in that** the image generation for the visualization of the magnetic resonance imaging is interrupted while the at least one ablation electrode (9) is fed with the MRI high-frequency signals (20).

7. Method according to one of claims 5 to 6, **characterized in that** the magnetic vortex field generated by the ablation current of the at least one ablation electrode (9) in the patient (6) is recorded via the magnetic resonance imaging system (2) and visualized as a current curve.

## Revendications

1. Système hybride (1) pour la réalisation d'une imagerie par résonance magnétique (IRM) et d'une ablation par radiofréquence sur un patient (6), comprenant les éléments suivants :
a) le système hybride (1) présente un système d'imagerie par résonance magnétique (2) dans lequel des signaux IRM haute fréquence (20) peuvent être générés pour la réalisation de l'imagerie par résonance magnétique et peuvent être fournis à une connexion de sortie (8) du système d'imagerie par résonance magnétique (2),
b) le système hybride (1) présente au moins une électrode d'ablation (9) pour la réalisation de l'ablation par radiofréquence,
c) ladite au moins une électrode d'ablation (9) est couplée à la connexion de sortie (8) du système d'imagerie par résonance magnétique (2), de sorte que l'ablation par radiofréquence peut être réalisée par l'intermédiaire de ladite au moins une électrode d'ablation (9) au moyen des signaux IRM haute fréquence (20),
**caractérisé en ce que**
le système hybride (1) est conçu pour amener les signaux IRM haute fréquence (20) soit à ladite au moins une électrode d'ablation (9) soit à une bobine d'émission IRM du système d'imagerie par résonance magnétique.

2. Système hybride selon la revendication 1,
**caractérisé en ce que** le système hybride (1) présente un circuit de génération d'impulsions par lequel les signaux haute fréquence nécessaires pour l'ablation par radiofréquence peuvent être amenés par impulsions à ladite au moins une électrode d'ablation (9).

3. Système hybride selon l'une des revendications précédentes, **caractérisé en ce que** ladite au moins une électrode d'ablation (9) est alimentée en un signal haute fréquence à la fréquence de Larmor.

4. Système hybride selon l'une des revendications précédentes, **caractérisé en ce que** le système hybride (1) est conçu pour enregistrer et visualiser le courant d'ablation, injecté dans le patient (6) par l'électrode d'ablation (9), par l'intermédiaire de l'imagerie (3) du système d'imagerie par résonance magnétique (2).

5. Procédé de fonctionnement d'un système hybride selon l'une des revendications précédentes,
**caractérisé en ce que** les signaux IRM haute fréquence (20) du système d'imagerie par résonance magnétique (2) sont amenés au moins temporairement à ladite au moins une électrode d'ablation (9), et
**en ce que** les signaux IRM haute fréquence (20) du système d'imagerie par résonance magnétique (2) sont amenés alternativement à ladite au moins une électrode d'ablation (9) et à une bobine d'émission IRM du système d'imagerie par résonance magnétique (2).

6. Procédé selon la revendication 5,
**caractérisé en ce que** la génération d'images pour la visualisation de l'imagerie par résonance magnétique est interrompue pendant que ladite au moins une électrode d'ablation (9) est alimentée en signaux IRM haute fréquence (20).

7. Procédé selon l'une des revendications 5 à 6,
**caractérisé en ce que** le champ magnétique tourbillonnaire généré dans le patient (6) par le courant d'ablation de ladite au moins une électrode d'ablation (9) est enregistré par le système d'imagerie par résonance magnétique (2) et est visualisé sous forme de courbe de courant.
